(19) Europäisches Patentamt
European Patent Office
Office européen des brevets

(11) Numéro de publication: **0 153 896 B1**

(12) # FASCICULE DE BREVET EUROPEEN

(45) Date de publication de fascicule du brevet: **27.05.92**

(51) Int. Cl.5: **A61K 9/22**, A61L 17/00, C08L 89/06

(21) Numéro de dépôt: **85420026.8**

(22) Date de dépôt: **19.02.85**

(54) **Réservoirs biocompatibles implantables à base de collagène permettant la conservation et/ou la culture cellulaires et/ou la libération contrôlée de principes actifs.**

(30) Priorité: **21.02.84 FR 8403180**

(43) Date de publication de la demande:
**04.09.85 Bulletin 85/36**

(45) Mention de la délivrance du brevet:
**27.05.92 Bulletin 92/22**

(84) Etats contractants désignés:
**AT BE CH DE FR GB IT LI LU NL SE**

(56) Documents cités:
**EP-A- 0 080 956**
**DE-A- 2 140 557**
**FR-A- 2 438 479**
**EP-B- 156 740**
**FR-A- 2 235 133**
**US-A- 4 351 337**

**CHEMICAL ABSTRACTS, vol. 96, no. 20, mai 1982, page 404, réf.no. 168742z, Columbus, Ohio, US; & JP - A - 82 09 711 (JAPAN ATOMIC ENERGY RESEARCH INSTITUTE) 19-01-1982**

(73) Titulaire: **BIOETICA, Société Anonyme**
**32 rue Saint Jean de Dieu**
**F-69007 Lyon(FR)**

(72) Inventeur: **Le Pivert, Docteur**
**2 rue Verdi**
**Nice (Alpes Maritimes)(FR)**
Inventeur: **Huc, Alain**
**96 Chemin des Fonds**
**Sainte-Foy-Les-Lyon (Rhône)(FR)**
Inventeur: **Gimeno, René**
**Métrieux - Chuyer**
**Pelussin (Loire)(FR)**

(74) Mandataire: **Maureau, Philippe et al**
**Cabinet Germain & Maureau Le Britannia - Tour C 20, bld Eugène Déruelle**
**F-69003 Lyon(FR)**

**EP 0 153 896 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des réservoirs biocompatibles implantables à base de collagène permettant la conservation et/ou la culture cellulaires et/ou la libération contrôlée de principes actifs.

On sait qu'il est couramment nécessaire en thérapeutique animale ou humaine ainsi qu'en biologie cellulaire de disposer de réservoirs susceptibles de conserver, concentrer et/ou distribuer, à vitesse contrôlée, "in vitro" et/ou "in vivo" une ou plusieurs substances actives et, le cas échéant, de créer "in situ" un nouveau produit.

On a également besoin d'enceintes de conservation et/ou de cultures "in vitro" et/ou "in vivo" de cellules, vivantes ou mortes, de bactéries, de virus...

Ces réservoirs sont destinés à jouer dans certains cas le rôle de conteneurs simples ou de conteneurs à parois actives, le lieu d'implantation et la fonction souhaitée imposant des formes et des biodégradabilités très diverses. De façon plus générale enfin ils devraient pouvoir servir de partie captrice ou émettrice d'un système de régulation plus complexe de libération d'une ou plusieurs substances actives (Biocapteur ou Bioréacteur) (BIOFUTUR, janvier 1984, Biofutur S.A. Ed. 75007 PARIS).

On connaît déjà des conteneurs en matière plastique implantables, notamment ceux du type "micro-pompe" qui permettent de faire passer dans l'organisme, à vitesse contrôlée, des doses déterminées d'une substance active. Il faut toutefois noter que les conteneurs de ce type ne sont que très partiellement biocompatibles et ne sont pas biodégradables.

On a établi par ailleurs (HUC A. et COMTE Ph. 1983, Biofutur, N° 9, 53-55) que le collagène constitue une substance de choix pour la réalisation de biomatériaux. Cette protéine, qui constitue la trame de soutien des tissus conjonctifs, présente, outre des propriétés mécaniques remarquables, une excellente biocompatibilité et une biodégradabilité éventuellement modifiable en fonction du but recherché.

La macromolécule de collagène d'une longueur de 3 000 Å et d'une largeur de 15 Å est constituée de trois chaînes peptidiques. Chaque chaîne, d'une masse de 100 000 daltons, est en forme hélicoïdale. Les axes des hélices s'enroulent en hélice autour d'un axe commun à l'intérieur d'une même macromolécule. Entre certaines chaînes peptidiques existent des liaisons réticulaires. L'arrangement ordonné des macromolécules entre elles conduit à la formation de fibres.

Les excellentes propriétés mécaniques du collagène proviennent, en grande partie, de la structure hélicoïdale et des liaisons réticulaires.

Le caractère antigénique du collagène est très faible. De ce fait, le collagène d'origine animale, ne provoque pas de réaction de rejet chez l'être humain, ainsi qu'il ressort d'une étude de TAKEDA, U. et coll. dans le Jal of Toxicology Sciences, Vol 7, suppl. II, 1982 pp 63-91.

On sait également qu'il est possible de modifier la perméabilité d'une membrane de collagène par des traitements divers (STENZEL K.H. et Coll. (1974) Ann. Rev. Biophys and Bioeng, 3, 231-253).

Toutes ces considérations ont conduit les inventeurs à étudier la possibilité qu'offrait le collagène pour la réalisation de réservoirs entièrement ou partiellement biodégradables éventuellement implantables dans l'organisme et susceptibles d'assurer la distribution contrôlée aussi bien que la rétention ou la concentration d'une substance active.

L'invention a donc pour objet des réservoirs biocompatibles implantables permettant la conservation et/ou la culture cellulaires et/ou la libération contrôlée de principes actifs et qui sont essentiellement constitués d'une enveloppe continue de collagène seul ou de collagène associé à des mucopolysaccharides et/ou à de la gélatine ; ces réservoirs peuvent ainsi renfermer une substance active sans que celle-ci soit mélangée au collagène.

Le procédé de fabrication des réservoirs biocompatibles à base de collagène selon l'invention va maintenant être décrit en détail.

La peau provenant de veau fraîchement abattu est lavée à l'eau. Les poils et les tissus sous-cutanés sont éliminés mécaniquement à l'aide d'une refendeuse et seul le derme est conservé. Celui-ci est alors haché et broyé. Le broyat obtenu est ensuite lavé par du tampon phosphate à pH 7,8 puis rincé à l'eau permutée. Il est alors placé dans une solution d'acide acétique à pH 3,5. La dilution doit être telle que la concentration en collagène soit de 1,5 %. L'ensemble est homogénéisé par ultra-sons, puis dégazé par agitation sous vide. Le gel ainsi obtenu est transformé en film et notamment selon les procédés décrits dans les brevets français 1 596 789 et 1 596 790 ou en tube par tout procédé connu en soi et notamment par le procédé revendiqué dans la demande de brevet Européen EP-B-156740 parallèle déposée ce même jour pour un "Procédé de fabrication de tubes de collagène notamment de tubes de faibles diamètres..." et qui comporte les étapes suivantes : extrusion dans une filière cylindrique équipée d'un tube central concentrique destiné à recevoir une partie du bain de coagulation, d'un gel acide aqueux contenant 1,5 % environ de collagène natif, suivie d'une coagulation des parois interne et externe du tube sortant de la filière

dans un bain de coagulation constitué d'environ 70 % d'acétone pour 30 % d'ammoniaque, et d'un séchage. La soudure des parties libres du film ou du tube est assurée par serrage entre deux machoires chauffantes à une pression de 500g/cm2 et à une température de 145 ° C pendant cinq secondes. Les caractéristiques mécaniques de la soudure obtenue sont les suivantes : force à l'arrachement de 400 g/cm pour une distance de recouvrement de 3 mm.

On obtient des réservoirs ayant la forme de coussins rectangulaires à partir de deux films plans de mêmes dimensions soudés sur les quatre côtés. Des conteneurs cylindriques sont obtenus par soudure des extrémités de tube. On peut également obtenir des réservoirs de formes diverses par moulage à chaud (vers 60 ° C) de films de collagène suivi de soudage de leurs bords.

En partant de gels homogènes collagène-mucopolysaccharides tels que ceux obtenus selon le brevet français 2 517 315, on obtient également des tubes ou des films pouvant être transformés en réservoirs comme dit plus haut, et qui présentent, par rapport aux mêmes éléments réalisés en collagène seul, une conductivité accrue.

Dans les cas où les réservoirs doivent être dotés de propriétés mécaniques très importantes, on peut associer le collagène à d'autres matériaux, et notamment à des polymères synthétiques. C'est ainsi que l'on peut renforcer des films de collagène par dépôt d'une trame, synthétique ou métallique, sur une couche de gel collagénique puis par recouvrement de l'ensemble obtenu d'une nouvelle couche de gel collagénique. Le sandwich ainsi constitué est séché dans un tunnel, dans les conditions mentionnées dans les brevets français 1 596 789 et 1 596 790.

Le matériau composite ainsi obtenu est alors soudé comme il a été dit précédemment pour constituer un réservoir. Des tubes en collagène renforcés peuvent être fabriqués en entourant un premier tube en collagène par la trame synthétique et en collant par dessus à l'aide d'une colle biologique type Tissucol® un deuxième tube de collagène d'un diamètre légèrement supérieur au premier. Le réservoir sera obtenu par soudure des parties libres du tube.

Les réservoirs biocompatibles ainsi obtenus peuvent être soumis à différents traitements afin, notamment, de modifier leur perméabilité, afin de pouvoir contrôler la diffusion de la substance active qu'ils doivent renfermer.

C'est ainsi que, si l'on désire diminuer la perméabilité de la membrane, améliorer sa biocompatibilité tout en augmentant sa résistance mécanique, on effectue une réticulation du collagène par traitement en étuve à 80 ° C pendant 48 heures sous un vide de 0,1 mm de mercure.

On peut d'autre part désirer disposer de systèmes présentant une très faible biodégradabilité. Selon les techniques utilisées jusqu'alors pour arriver à ce but, on tanne le collagène en fixant différentes substances, en particulier des aldéhydes, sur les groupements actifs de la protéine ; le collagène perd, de ce fait, une partie de ses propriétés biologiques, mais ce traitement risque d'entraîner in vivo une calcification du matériau collagénique ; de plus, les substances fixées sur les groupements actifs risquent de se relarguer et d'avoir un effet néfaste sur l'organisme.

Selon la présente invention, on bloque les groupements réactifs du collagène, sans apport de substances étrangères (à la différence du procédé décrit et revendiqué dans le brevet français FR-A-2 235 133) susceptibles de diffuser dans l'organisme, par introduction de groupements azides dans la molécule de collagène selon le processus suivant :

On place le matériau pendant huit jours dans une solution méthanolique acide (méthanol pur, concentration en HCl de 0,3 N). Il se produit une méthylation des groupements acides libres du collagène selon le schéma suivant :

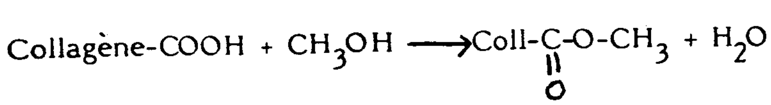

Les groupements acides sont ceux des acides aspartique et glumatique.

Le matériau est ensuite lavé abondamment à l'eau puis placé dans une solution aqueuse à 1 % d'hydrazine ; les groupements méthylés se transforment en groupements hydrazides selon la réaction :

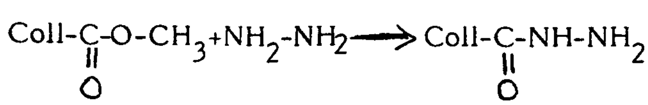

Une durée de quatre à cinq heures est nécessaire pour obtenir le nombre maximum de groupements

transformés. Le matériau est alors rincé abondamment à l'eau, puis soumis à l'action d'une solution de nitrite de sodium dans l'acide chlorhydrique 0,3 N. On obtient alors les groupements azides selon la réaction :

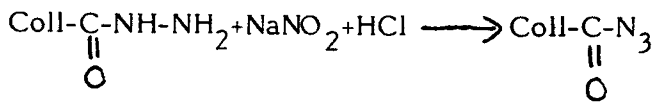

La durée totale de cette réaction est d'environ cinq minutes.

Le matériau est ensuite placé pendant deux heures dans du tampon borate à pH9 (acide borique, tétraborate de sodium 0,2 M) puis rincé abondamment à l'eau.

Toutes les étapes du procédé sont réalisées à température ambiante (20 ° C environ).

Les dosages effectués sur le matériau final montrent qu'il ne contient ni hydrazine, ni nitrite de sodium, ni aucune autre substance étrangère.

La calorimétrie différentielle programmée révèle que la température de dénaturation du collagène est de 10 ° C plus élevée pour du collagène ainsi traité que pour du collagène brut ou simplement réticulé.

Le collagène activé est donc plus stable que le collagène reconstitué ; la température de dénaturation dépasse alors 37 ° C, température de l'organisme. Le collagène conserve toute sa structure native et, par là, l'essentiel de ses propriétés, en particulier mécaniques. De plus, il résiste beaucoup mieux à l'attaque des enzymes protéolytiques.

Un film de collagène ainsi traité a été implanté chez le rat, en situation intrapéritonéale et en situation sous-cutanée. Son comportement a été comparé à celui d'un film de collagène placé dans les mêmes situations.

Les résultats des examens histologiques font ressortir :

- qu'au bout de 21 jours, le film brut a disparu alors que, au bout de 90 jours, le film activé est toujours identifiable ;
- que des cellules mésothéliales existent sur les deux implants, mais que leur apparition est retardée sur le matériau activé et qu'elles s'y développent moins bien.

Afin de diminuer la perméabilité du collagène pour ralentir la diffusion du principe actif, une grosse molécule peut être greffée à la surface du collagène. Plus la taille de cette molécule est importante, plus elle joue le rôle de barrière pour le cheminement d'une substance à travers la membrane du collagène.

Selon un autre mode de réalisation de l'invention, on greffe au collagène une enzyme susceptible d'agir "in vivo" sur la substance que le système est destiné à recevoir. L'organisme aura ainsi à sa disposition une nouvelle substance préparée ex-temporanément.

Ce procédé de greffage peut, par exemple, être celui décrit dans le brevet français FR-A-2 235 133 qui consiste à immerger le produit collagénique dans un mélange méthanol-acide pendant plusieurs heures à température ambiante, à rincer le produit à l'eau; puis à le plonger dans un bain d'hydrazine pendant plusieurs heures, à traiter le produit obtenu par un mélange nitrite de sodium-acide pendant quelques minutes, puis à le laver au chlorure de sodium. Le produit collagénique ainsi activé est immergé dans une solution enzymatique à pH 9 pendant environ deux heures pour former un support collagénique enzymatiquement actif.

On peut accroître de façon sensible la conductivité en réalisant une paroi constituée de deux couches de collagène prenant en sandwich une mince couche de carbone finement divisée.

Les réservoirs biocompatibles peuvent enfin être stérilisés par une dose de 2,5 MRads de rayonnements $\gamma$. Un tel traitement inactive définitivement tous les microorganismes, réticule le collagène et inhibe son action sur la coagulation du sang.

Les matériaux ainsi obtenus sont analysés sur trois plans : sur le plan chimique, sur le plan des masses moléculaires et sur le plan de la structure (HUC A. et BARTHOLIN F. (1978) Revue Institut Pasteur Lyon, 11, N° 2,179-190).

Le premier groupe d'analyses met en évidence le degré de pureté du collagène, le deuxième l'intégrité des chaînes peptidiques et le troisième la non destruction de la structure hélicoïdale. Toutes ces analyses démontrent que la protéine possède toutes les caractéristiques du collagène.

De plus, la calorimétrie différentielle programmée permet de déterminer la thermostabilité de la structure native et par là de connaître la biodégradabilité du biomatériau.

Les résultats obtenus sont les suivants :

Perméabilité :

Les études de perméabilité ont été effectuées sur une solution de glycérol à 20 % placée dans un tube de collagène dont les parois avaient une épaisseur de 100 µm. Pour cela, du glycérol marque au carbone 14 a été dilué dans le glycérol non marqué qui a servi à la préparation de la solution. Le tube utilisé avait été réticulé par la chaleur comme indiqué plus haut et la quantité de solution utilisée était de 500 µl.

Le réservoir ainsi préparé a été mis dans 10 ml d'eau. La diffusion a été suivie en mesurant à des intervalles de temps connus la radioactivité du bain entourant le réservoir. La courbe d'évolution de cette radioactivité en C.P.M. de 200 µl de bain entourant le réservoir en fonction du temps est donnée sur la figure 1. On peut constater que même avec le glycérol, petite molécule très soluble dans l'eau, deux heures au moins sont nécessaires pour obtenir l'équilibre entre l'intérieur et l'extérieur du tube. Le réservoir de collagène amène donc un effet retard net dans la diffusion de la substance qu'il contient.

Biodégradabilité

Les expériences effectuées sur des films de collagène tannés par la méthode aux azides ont montré que la température de début de dénaturation était augmentée de 10 °C par rapport à un collagène brut, cette température passant de 35 à 45 °C. Dans ces conditions, le collagène "in vivo" ne perdra pas sa structure hélicoïdale et conservera ses propriétés mécaniques.

De plus, il s'est avéré qu'un réservoir de collagène implanté en position sous-cutanée n'était pas digéré par les collagénases au bout de 90 jours. Après trois semaines, un réservoir en collagène pur a complètement disparu. Enfin, une biodégradabilité plus ou moins importante pourra être obtenue en modifiant le temps de contact du collagène avec l'alcool méthylique dans la première étape de la méthode de traitement aux azides. Un temps de contact plus faible entraînera une diminution du nombre de groupements acides bloqués et, par là même, une protection moindre du collagène. La biodégradabilité du réservoir sera donc plus importante que dans le cas du traitement complet.

Modification de la perméabilité

Elle est obtenue en faisant varier l'épaisseur des parois du collagène, en préparant des conteneurs à double paroi ou en greffant des grosses molécules à la protéine.

Présentation des substances actives

Elles peuvent être mises à l'intérieur du réservoir sous forme de solutions ou de liquides purs. Si une diffusion plus lente est désirée, il est préférable de les présenter sous forme de poudre ou de lyophilisat.

Pour obtenir une diffusion encore plus lente, les poudres peuvent être encapsulées dans du collagène selon le procédé décrit dans la demande de brevet français FR-A-2 524 471. La substance peut être incluse dans une éponge de collagène elle-même placée dans un réservoir.

Le procédé exposé ci-dessus conduit donc à l'obtention de réservoirs possèdant dans l'organisme des vitesses de dégradations variables et laissant diffuser plus ou moins rapidement les substances dans l'organisme. Ces deux paramètres seront fixés par l'épaisseur, la texture des parois et par les présentations des principes actifs dans le réservoir.

Le conditionnement des réservoirs biocompatibles à base de collagène selon l'invention n'est nullement limité aux films et aux tubes ou conteneurs et il est évident que l'invention embrasse également d'autres forme de ces systèmes, tels que des billes, capsules...dans laquelle ils sont susceptibles de remplir les fonctions décrites ci-avant.

Les applications des réservoirs biocompatibles selon la présente invention sont nombreuses et variées ; c'est ainsi que ces réservoirs partiellement ou totalement biodégradables (éventuellement implantables in-vivo) peuvent intervenir - isolément ou en association - avec d'autres systèmes capteurs et/ou régulateurs.

On sait que la voie percutanée présente des avantages incontestables dans les traitements à visée systémique mais que l'emploi des formes galéniques classiques, visant à une action prolongée s'accorde assez mal avec ce mode d'administration. Les problèmes majeurs rencontrés sont :

- la difficulté d'appliquer une posologie précise et la médiocre maniabilité des formes pâteuses ou liquides, ce qui est gênant pour le malade, surtout en ambulatoire ;
- le difficile respect de la surface d'application, dont dépend le taux de pénétration ;
- le rôle important du mode d'application, puisqu'une friction de la peau peut augmenter la résorption par vasodilatation locale.

La mise au point des réservoirs biocompatibles à base de collagène selon l'invention permet de disposer de systèmes transdermiques solides, à libération contrôlée de principe actif. Le principe actif,

exactement dosé, dispersé dans une phase liquide, diffuse à une vitesse constante, prédéterminée "in vitro" pendant plusieurs jours à travers la paroi de collagène jouant le rôle de membrane poreuse semi-perméable ; on efface ainsi les variabilités inter et intra-individuelles dues notamment à l'âge de la peau et au lieu d'application.

Le réservoir biocompatible selon l'invention peut ainsi se présenter sous forme d'une pastille creuse composite (du type appelé "Tube-patch") dont l'une des parties est fixable à la peau, l'autre constituant la surface active en contact avec la peau.

Parmi les très nombreux principes actifs qu'il est possible de libérer à l'aide des réservoirs biocompatibles à base de collagène selon l'invention, on peut citer la Trinitrine, la Scopolamine, l'Ephédrine, les produits anti-inflammatoires, les cicatrisants, décapants, antibiotiques, anesthésiques ou analgésiques sans que pour autant cette liste soit limitative.

Les réservoirs biocompatibles selon l'invention peuvent également être appliqués de façon non limitative par voie intracavitaire : vagin, utérus, plaies opératoires par exemple sous forme d'implant intratumoral (chimiothérapie "in situ" par exemple).

Il se révèlent aussi fort intéressants par voie intravasculaire, par exemple pour l'induction des scléroses veineuses, le principe actif pouvant être alors une solution glucosée à pourcentage variable et fonction de l'effet que l'on cherche à obtenir sur l'intima. On peut ainsi, avec un pourcentage inférieur d'agent sclérosant, obtenir un effet sclérosant plus sûr en raison du contact prolongé avec la paroi avec, comme avantage supplémentaire, la biodégradabilité et l'absence de toxicité du matériau.

L'utilisation des réservoirs biocompatibles selon l'invention a la réalisation de pancréas artificiel est aussi très pleine de promesses puisqu'on peut enfermer dans une structure imperméable aux anticorps et aux lymphocytes, mais perméable à l'insuline et au glucose, des cellules allogéniques vivantes et fonctionnelles.

Une autre voie d'utilisation des réservoirs biocompatibles selon l'invention est celle de conteneur et de micro-implants de cellules vivantes.

Utilisés comme conteneurs de cellules pour la congélation et/ou la lyophilisation, ils permettent d'effectuer ces traitements sur des cellules vivantes préalablement extirpées de tissus sains ou de tumeurs, ou sur des cellules cultivées.

La paroi de ces conteneurs biodégradables plus ou moins rapidement permet, après implantation "in vivo", la libération soit de principes actifs, soit des cellules elles-même, la perméabilité assurant la nutrition "in vivo" (cellules pancréatiques, thyroïdiennes, ostéoblastes, cellules sanguines, placentaires, spermatozoïdes, ovules, etc...)

Ces conteneurs peuvent donc servir de récipients au cours du prélèvement, de la conservation, du transport et de la transplantation de cellules viables ; ils protègent leur contenu contre les attaques de l'organisme tout en permettant le déversement de principes actifs dans cet organisme.

Ils sont enfin susceptibles de servir de capteurs sélectifs de substances biologiques (fixation sélective d'antigènes ou anticorps).

Il est bien évident que le champ ouvert aux réservoirs biocompatibles selon l'invention est très vaste puisqu'il est possible avec les éléments constants de ces systèmes : collagène seul ou associé à des mucopolysaccharides et/ou à des gélatines présentant une perméabilité membranaire et une biodégradabilité fixée à la fabrication, de jouer sur de nombreuses variables, parmi lesquelles se trouvent la taille et la forme du conteneur, le type de principe actif et le lieu d'implantation.

**Revendications**

1. Réservoir biocompatible à base de collagène, constitué par une enveloppe continue d'un film de collagène, caractérisé en ce qu'il comprend un premier tube en collagène entouré par une trame synthétique, et un deuxième tube de collagène de diamètre supérieur, les parties libres des tubes étant soudées.

2. Réservoir biocompatible à base de collagène, constitué par une enveloppe d'un film de collagène, selon la revendication 1, caractérisé en ce qu'il contient une substance active incluse dans une éponge de collagène, elle-même placée dans l'enveloppe.

3. Procédé de fabrication de réservoirs biocompatibles à base de collagène, selon la revendication 1, selon lequel on part d'un gel aqueux de collagène natif, on extrude dans une filière un film à partir du gel aqueux, on coagule le film dans un bain de coagulation, on sèche le film coagulé, **caractérisé en ce qu'**on soude les côte libres de deux films par serrage entre deux machoires chauffantes.

4. Procédé de fabrication selon la revendication 3, caractérisé en ce qu'on effectue une réticulation par la chaleur du film de collagène ou du réservoir, par un traitement en étuve sous vide.

5. Pancréas artificiel, comprenant un réservoir selon l'une quelconque des revendications 1 et 2, caractérisé en ce que ledit réservoir enferme des cellules allogéniques vivantes et fonctionnelles.

6. Conteneur de cellules pour la congélation et/ou la lyophilisation, comprenant un réservoir selon l'une quelconque des revendications 1 et 2.

## Claims

1. Collagen-based biocompatible reservoir consisting of a continuous envelope of a collagen film, characterised in that it comprises a first collagen tube surrounded by a synthetic matrix, and a second collagen tube of greater diameter, the free parts of the tubes being bonded.

2. Collagen-based biocompatible reservoir, consisting of a collagen-film envelope according to Claim 1, characterised in that it contains an active substance enclosed in a collagen sponge, which is itself placed in the envelope.

3. Process for manufacturing collagen-based biocompatible reservoirs according to Claim 1, according to which an aqueous gel of native collagen is used as the starting material, a film is extruded in a die from the aqueous gel, the film is coagulated in a coagulation bath, and the coagulated film is dried, characterised in that the free sides of the two films are bonded by pressing between two heating jaws.

4. Manufacturing process according to Claim 3, characterised in that a crosslinking is performed by heating the collagen film or the reservoir, by treatment in an oven under vacuum.

5. Artificial pancreas comprising a reservoir according to any one of Claims 1 and 2, characterised in that the said reservoir contains living and functional allogeneic cells.

6. Cell container for freezing and/or freeze-drying, comprising a reservoir according to any one of Claims 1 and 2.

## Patentansprüche

1. Biologisch verträglicher Behälter auf Basis von Kollagen, gebildet durch eine durchgehende Umhüllung aus einer Kollagenfolie, dadurch gekennzeichnet, daß er ein erstes Rohr aus Kollagen aufweist, das von einem synthetischen Gerüst und von einem zweiten Rohr aus Kollagen mit größerem Durchmesser umrundet ist, wobei die freien Enden der Rohre verschweißt sind.

2. Biologisch verträglicher Behälter auf Basis von Kollagen, gebildet durch eine Umhüllung aus einer Kollagenfolie nach Anspruch 1, dadurch gekennzeichnet, daß er einen Wirkstoff enthält, der in einem Kollagenschwamm eingeschlossen ist, der selbst wiederum in der Umhüllung angeordnet ist.

3. Verfahren zum Herstellen von biologisch verträglichen Behältern auf Basis von Kollagen nach Anspruch 1, bei dem von einem wässrigen Gel an natürlichem Kollagen ausgegangen wird, in einem Spritzgußmundstück eine Folie aus diesem wässrigen Gel extrudiert wird, die Folie in einem Koagulationsbad koaguliert wird, sowie die koagulierte Folie getrocknet wird, dadurch gekennzeichnet, daß die freien Enden von zwei Folien durch Zusammendrücken zwischen zwei Heizstempeln verschweißt werden.

4. Herstellungsverfahren nach Anspruch 3, dadurch gekennzeichnet, daß eine Vernetzung der Kollagenfolie oder des Behälters durch Wärme mittels einer Behandlung in einem Trockenofen im Vakuum durchgeführt wird.

5. Künstlicher Pankreas, enthaltend ein Behälter nach einem der Ansprüche 1 oder 2, dadurch gekennzeichnet, daß der Behälter lebende und funktionelle allogene Zellen einschließt.

6. Behältnis für Zellen zum Eindicken und/oder zur Lypophilisierung, enthaltend einen Behälter nach

einem der Ansprüche 1 oder 2.

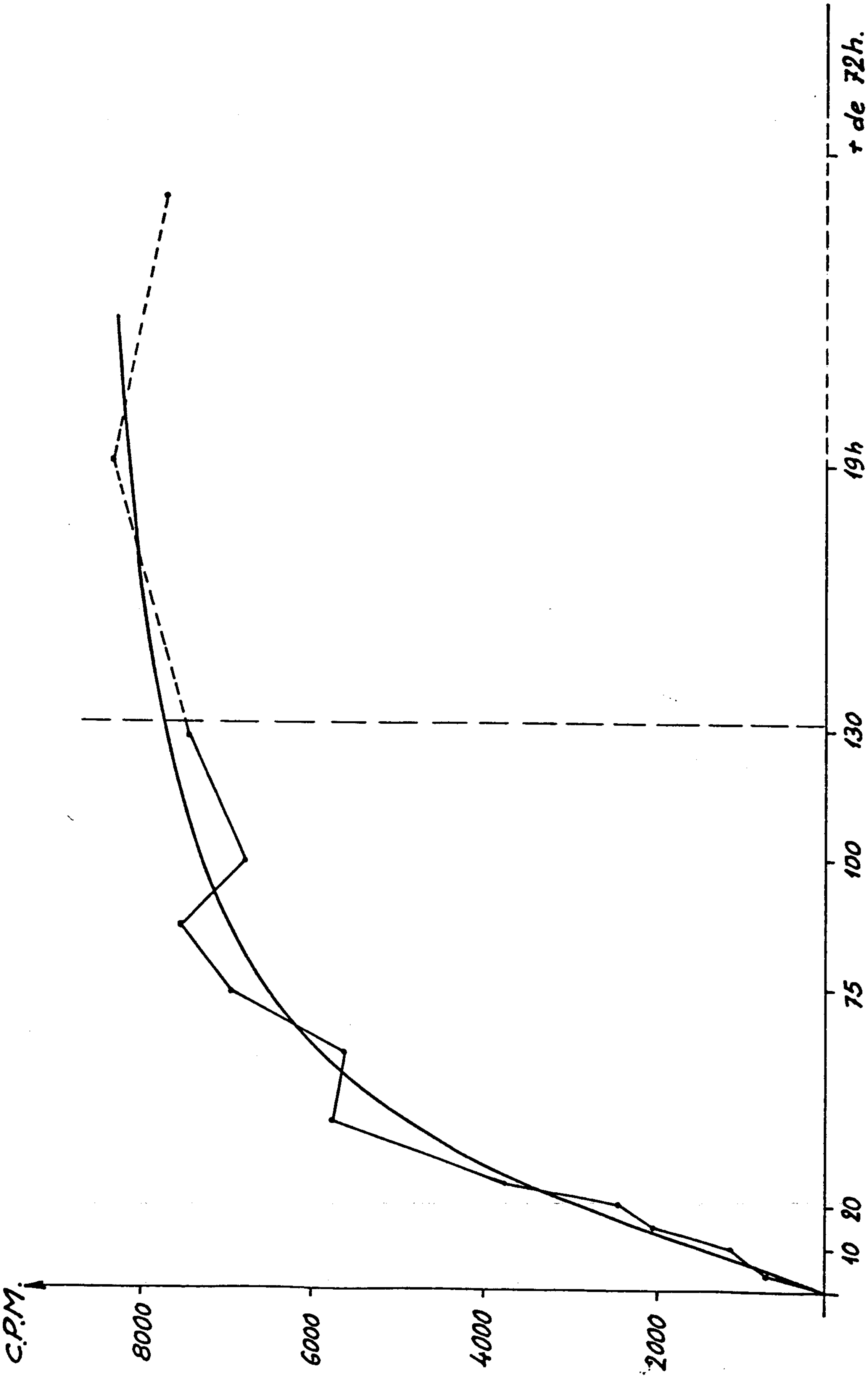
C.P.M.
8000
6000
4000
2000
10
20
75
100
130
mn
19h
+ de 72h.